(19) Europäisches Patentamt
European Patent Office
Office européen des brevets

(11) EP 2 334 084 A1

(12) # EUROPEAN PATENT APPLICATION

(43) Date of publication:
15.06.2011 Bulletin 2011/24

(51) Int Cl.:
*H04N 9/04 (2006.01)* *A61B 1/04 (2006.01)*

(21) Application number: 09175523.1

(22) Date of filing: 10.11.2009

(84) Designated Contracting States:
AT BE BG CH CY CZ DE DK EE ES FI FR GB GR HR HU IE IS IT LI LT LU LV MC MK MT NL NO PL PT RO SE SI SK SM TR

(71) Applicant: Chung Shan Institute of Science and Technology
Taoyuan County 325 (TW)

(72) Inventors:
- Weng, Ping-Kuo
325 Taoyuan County (TW)
- Wu, Ying-Yih
516 Changhua County (TW)
- Wu, Sian-Ming
325 Taoyuan County (TW)

(74) Representative: Jordan, Volker Otto Wilhelm
Weickmann & Weickmann
Patentanwälte
Postfach 860 820
81635 München (DE)

(54) ## Image sensing device and system

(57) The invention provides an image sensing device capturing a gray level image and a chromatic image on a frame via a mixing illumination of a first light and a second light. The image sensing device includes a color filter array module and a controlling module. The color filter array module includes a first filter unit, a second filter unit, a third filter unit, and a fourth filter unit. The first filter unit is used to sense a first pixel data at the frame; the second filter unit is used to sense a second pixel data at the frame; the third filter unit is used to sense a third pixel data at the frame; and the fourth filter unit is used to sense a narrow banding data. The controlling module controls the sensing form of the color filter array module according to the first light or the second light.

6
60
620
62
622
624
R
B
R
B
626
NB
G
NB
G
R
B
R
B
NB
G
NB
G
66
controlling module
64
luminous module
68

FIG. 3

EP 2 334 084 A1

## Description

### 1. Field of the invention

The present invention generally relates to an image sensing device, and more particularly, the image sensing device and system of the invention is capable of capturing a chromatic image and a gray level image on a frame synchronously.

### 2. Description of the prior art

Endoscope systems are generally used for medical treatment. The conventional endoscope device performs a shooting by invading a body directly, and it usually makes the patient feel uncomfortable. In recent years, with the development of technology and the progress of the integrated circuit process and the wireless transmission technology, the volume of an image sensor has been gradually reduced to form an endoscope in a capsule. Since the capsule endoscope can perform the shooting along gullet toward small intestine, the examiner can shoot the parts of the possible pathological changes in the patient body via controlling the wireless capsule endoscope, and judges the causes of the possible pathological changes according to the images. Accordingly, the defects of the conventional invading endoscope can be improved.

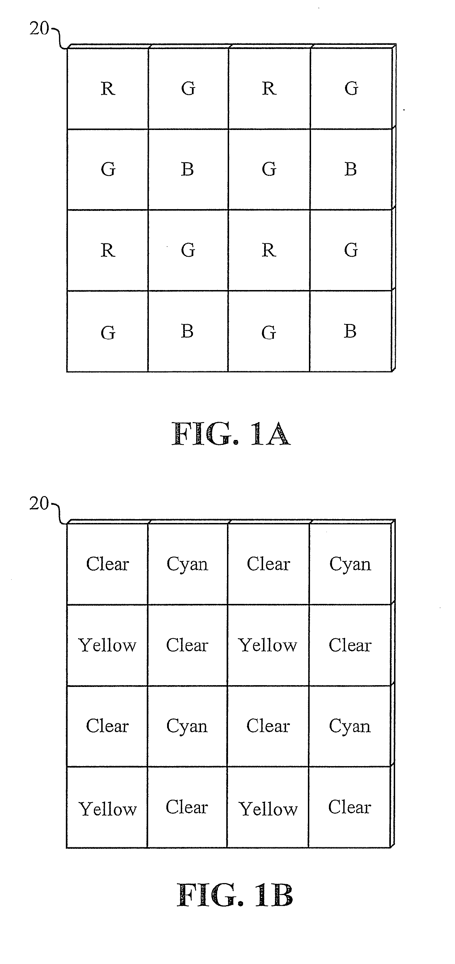

However, the image sensor of the general capsule endoscope is mainly used for getting the chromatic image. Please refer to FIG. 1A~1D. FIG. 1A~1D illustrate the color filter module 20 of the prior art respectively. As shown in FIG. 1A, the array of the color filter module 20 is the most commonly used array at present; the color filter module 20 includes a clear pixel, a yellow pixel, and a cyan pixel. Practically, the clear pixel includes a red pixel, a green pixel, and a blue pixel; the yellow pixel includes a red pixel and a blue pixel; the cyan pixel includes a green pixel and a blue pixel; the original red (R) pixel, the original green (G) pixel, and the original blue (B) pixel are generated by the variation of adjacent pixel intensity; finally, the original pixels are reduced to the chromatic image.

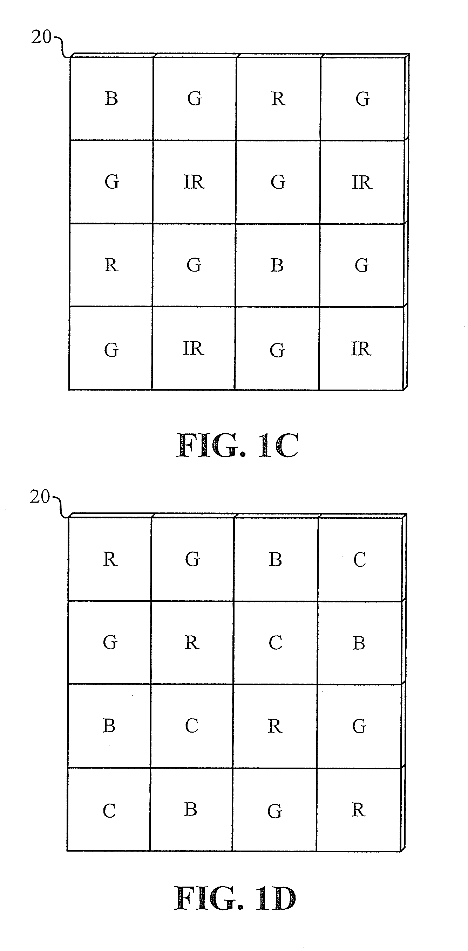

The color filter module 20 increases an infrared ray (IR) pixel in FIG. 1C. It can clear the IR pixel of other RGB pixels via the electrical signal processing method; therefore, the system can reach the function without the IR capturing filter. In FIG. 1D, the cyan filter unit is added to the color filter module 20 to increase the color gamut of the chromatic image via a four-pixel array.

In practical applications, examiners usually discover that the chromatic image is unable to show the disease region clearly. That is because the primary cancer will grow on the vessel surface, and the hemoglobin has obvious absorption spectrum characteristics to the light with the wavelength of 415 nm and 540 nm. Although, the capsule endoscope can perform the shooting inside the small intestine presently, it only can shoot the chromatic image. As for the above-mentioned specific wave band image or narrow band image, they can not be captured so that the examiner can not make precise recognition according to the disease region.

### SUMMARY OF THE INVENTION

Accordingly, an aspect of the present invention is to provide an image sensing device, and the image sensing device is used to observe a chromatic image and a gray level image with high contrast on an object surface synchronously, so that an observer can not only observe the surface of the object according to the chromatic image, but also judge the unusual form of the object surface via the gray level image with high contrast.

According to an embodiment of the invention, the image sensing device of the invention is capable of capturing a gray level image and a chromatic image on a frame via a mixing illumination of a first light and a second light. The image sensing device includes a color filter array module and a controlling module. The color filter array module includes a first filter unit, a second filter unit, a third filter unit, and a fourth filter unit. The first filter unit is located in a first region and used for sensing a first pixel of the frame. The second filter unit is located in a second region and near the first region; the second filter unit is used for sensing a second pixel of the frame. The third filter unit is located in the third region and near the second region; the third filter unit is used for sensing a third pixel of the frame. The fourth filter unit is located in a fourth region and near the first region and the third region; the fourth filter unit is used for sensing a narrow band pixel of the frame.

In this embodiment, the controlling module is coupled to the color filter array module, the first light, and the second light; the controlling module is used for controlling a sensing form of the color filter array module according to the first light or the second light. When the first light performs exposure, the controlling module starts the fourth filter unit to sense the narrow band pixel and generates the gray level image with high contrast according to the narrow band pixel. Similarly, when the second light performs exposure, the controlling module starts the first filter unit, the second filter unit, and the third filter unit to sense the first pixel, the second pixel, and the third pixel, and generates the chromatic image according to the first pixel, the second pixel, and the third pixel.

According to another embodiment of the invention, the image sensing system of the invention includes an image sensing device and a data process device. The image sensing device includes a color filter array module, a luminous module, and a controlling module. The color filter array module is used to generate a first pixel, a second pixel, a third pixel, and a fourth pixel. The luminous module is used to generate a multi-band light or a narrow band light. The controlling module is coupled to the color filter array module and the luminous module, and the controlling module is used to control a sensing form of the color filter array module according to the multi-band light or the narrow band light by the luminous module. The data process device is coupled to the image sensing device, for receiving the first pixel, the second pixel, and the third pixel to reform the chromatic image, and/or receiving the fourth pixel and showing the gray level image.

To sum up, the image sensing device and system provided by the invention uses the filter unit array of the new-style color filter and controls the multi-band light and the narrow band light through the filter unit of the color filter to obtain each of band gray level images. Wherein, the gray level image of the red pixel, the green pixel, and the blue pixel can be reconstructed to be a full-band chromatic image, and the narrow band pixel will show the gray level image with high contrast. Therefore, the image sensing device and system of the invention can synchronously or independently show different corresponding band images by the outer display device.

The objective of the present invention will no doubt become obvious to those of ordinary skill in the art after reading the following detailed description of the preferred embodiment, which is illustrated in the various figures and drawings.

## BRIEF DESCRIPTION OF THE APPENDED DRAWINGS

FIG. 1A~1D illustrate the color filter module of the prior art respectively.

FIG. 2A illustrates a schematic diagram of the image sensing device of the embodiment of the invention.

FIG. 2B illustrates a schematic diagram of another array shape of the color filter array module in FIG. 2A.

FIG. 3 illustrates a schematic diagram of the image sensing system of another embodiment of the invention.

## DETAILED DESCRIPTION OF THE INVENTION

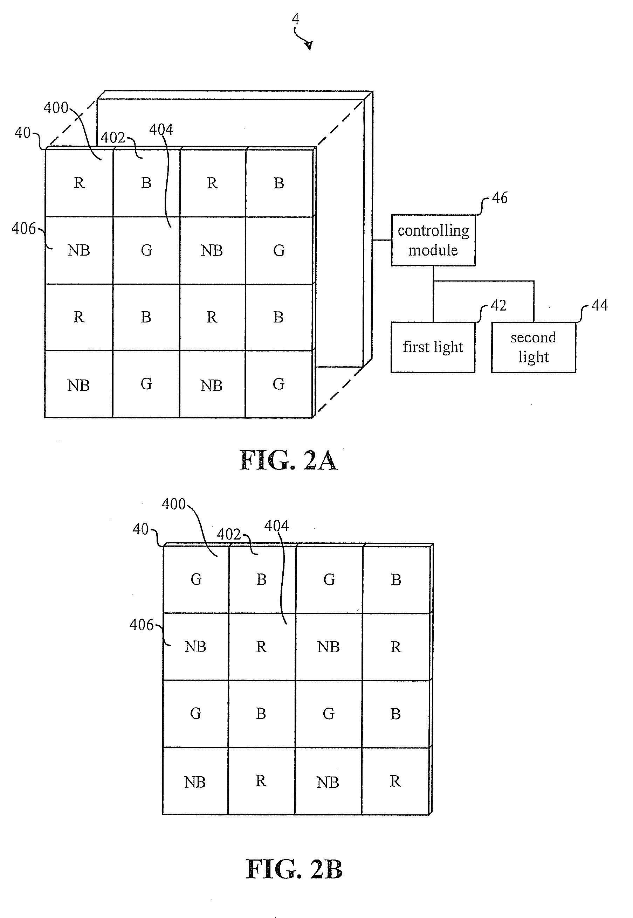

Please refer to FIG. 2A. FIG. 2A illustrates a schematic diagram of the image sensing device 4 of an embodiment of the invention. Practically, the image sensing device 4 of the invention can be a charge coupled device (CCD) or a complementary metal-oxide semiconductor (CMOS), but not limited to this.

In this embodiment, the image sensing device 4 of the invention includes a color filter array module 40 and a controlling module 46. The color filter array module 40 can be directly formed on the surface of the image sensing device, or the color filter array module 40 and the image sensing device 4 are assembled in a body to form the color filter array module 40.

As shown in FIG. 2A, the color filter array module 40 includes the first filter unit 400, a second filter unit 402, a third filter unit 404 and a fourth filter unit 406. The first filter unit 400 is located in a first region, and the first filter unit 400 is used to sense a first pixel of the frame. The second filter unit 402 is located in a second region and near the first region, and the second filter unit 402 is used to sense a second pixel of the frame. The third filter unit 404 is located in a third region and near the second region, and the third filter unit 404 is used to sense a third pixel of the frame. The fourth filter unit 406 is located in a fourth region and near between the first region and the third region, and the fourth filter unit 406 is used to sense a narrow band pixel of the frame.

In this embodiment, the red (R) pixel can only pass through the first filter unit 400, the blue (B) pixel can only pass through the first filter unit 402, and the green (G) pixel can only pass through the first filter unit 404. Therefore, the first pixel, the second pixel, and the third pixel can be defined to correspond to the R pixel, the B pixel, and the G pixel respectively. In practical applications, the first filter unit 400, the second filter unit 402, and the third filter unit 404 can be arranged as the array shown in FIG. 2B, but not limited to this.

It should be noticed that the fourth filter unit surface can be plated with a specific wavelength film or an electroless plating filter film. Practically, the fourth filter unit 406 surface can be plated with the 415nm wavelength filter film or the electroless plating filter film. If the fourth filter unit 406 surface is plated with the 415nm wavelength filter film, only the 415nm light can pass through the fourth filter unit 406 to form the gray level image with high contrast; similarly, if the fourth filter unit 406 is not plated with the filter film, the light can fully pass through the fourth filter unit 406 to form the gray level image.

In practical applications, the image sensing device 4 captures a gray level image and a chromatic image on a frame via a mixing illumination of a first light 42 and a second light 44. Wherein, the first light 42 is a narrow band light and the second light 44 is a white light, and the first light 42 and the second light 44 can be a suitable light emitting diode (LED).

When the mixing illumination is used, the first light will generate interference to RGB units of the color filter array module 40 to cause the shift of the sensing level. Therefore, in this embodiment, the following equations are used to perform the color compensation, wherein the penetration coefficient *($t_r$, $t_g$, and $t_b$)* are used to express the RGB pixels to the NB band respectively, and the value *(R*0, *G*0, and *B*0) are considered as RGB pixels respectively.

$$R = R0 - t_r \times (NB(R) + NB(L))/2$$

$$G = G0 - t_g \times (NB(U) + NB(D))/2$$

$$B = B0 - t_b \times (NB(RU) + NB(RD)) + NB(LU) + NB(LD))/4$$

In this embodiment, the controlling module 46 of the image sensing device 4 is coupled to the color filter array module 40, the first light 42, and the second light 44. The controlling module 46 can control a sensing form of the color filter array module 40 according to the first light 42 or the second light 44. Additionally, the controlling module 46 can control the sensing time luminescence or the crisscrossing way luminescence of the first light 42 and the second light 44 according the color filter array module 40. For example, the first light 42 is firstly turned on, and then the second light 44 is also turned on.

When the first light 42 performs exposure, the controlling module 46 starts the fourth filter unit 406 to sense the narrow band pixel and generates the gray level image with high contrast according to the narrow band pixel. In practical applications, the narrow band image technology can perform the inspection aiming at the gastrointestinal tumor in the human body, and the cell lesion less than 5 mm of the diameter will be recognized in the gastrointestinal system according to the narrow band light. Compared to the white light luminance, the narrow band light can help the observer inspect the primary cancer symptom more efficiently.

On the contrary, when the second light 44 performs exposure, the controlling module 46 starts the first filter unit 400, the second filter unit 402, and the third filter unit 404 to sense the first pixel, the second pixel, and the third pixel, and generates the chromatic image according to the first pixel, the second pixel, and the third pixel. Practically, the first pixel, the second pixel, and the third pixel can correspond to a red pixel, a blue pixel, and a green pixel respectively, but not limited to this.

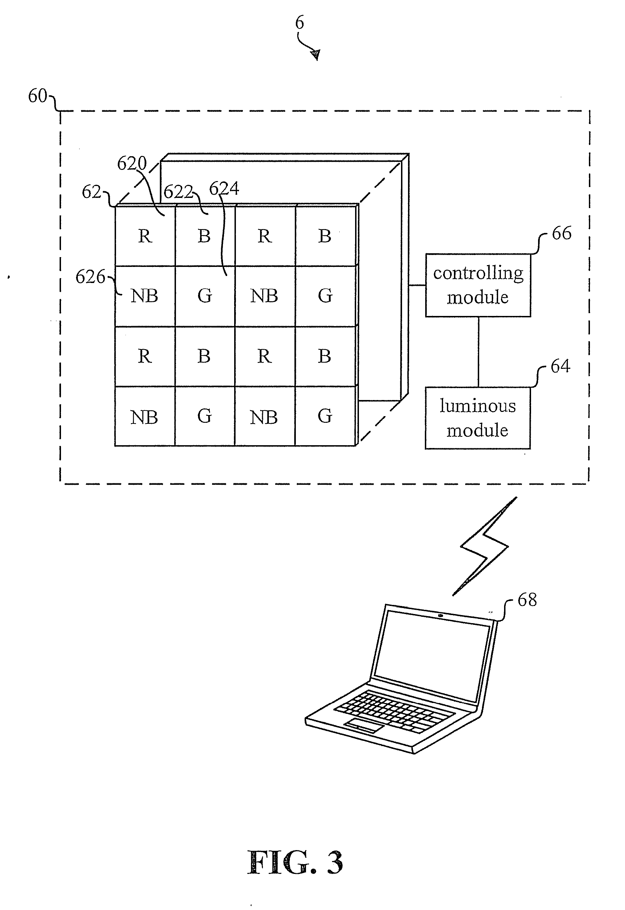

Please refer to FIG. 3. FIG. 3 illustrates a schematic diagram of the image sensing system 6 of another embodiment of the invention. As shown in FIG. 3, the image sensing system 6 includes an image sensing device 60 and a data process device 68. The image sensing device 60 is used to capture a gray level image and a chromatic image on a frame, and the image sensing device 60 includes a color filter array module 62, a luminous module 64, and a controlling module 66. Practically, the image sensing device 60 can be a charge coupled device (CCD) or a complementary metal-oxide semiconductor (CMOS), but not limited to this.

The color filter array module 62 is used to generate a first pixel, a second pixel, a third pixel, and a fourth pixel. In this embodiment, the structure of the color filter array module 62 and the above-mentioned color filter array module 40 are the same, so it will no longer be explained again.

The luminous module 64 is used for generating a multi-band light or a narrow band light, and the luminous module 64 is assembled by one or several light emitting diode (LED) with the color filter array module 62, or selecting the specific band light source according to the object property. Practically, the multi-band light is a white light, and the narrow band light is a specific band light, such as 415 nm or 540 nm of the band, etc. Generally, the light transparent capability of 415 nm band is not good, so it is suitably used to view the shallow lesion. On the contrary, the light transparent capability of 540nm band is good, so it can be used to view the depth vessel distribution.

The controlling module 66 is coupled to the color filter array module 62 and the luminous module 64. The controlling module 66 is used to control a sensing form of the color filter array module 62 according to the multi-band light or the narrow band light by the luminous module 64. The controlling method of controlling module 66 and the above-mentioned controlling module 46 is the same, so it will no longer be explained again.

The data process device 68 is used for receiving the first pixel, the second pixel, and the third pixel to reform the chromatic image, and/or receiving the fourth pixel and showing the gray level image. In practical applications, the chromatic image and the gray level image can be shown on the display of the data process device 68 synchronously, so that the examiner can judge whether the small intestine of the patient has any lesion according to the chromatic image and the gray level image different in the same frame. Additionally, the data process device 68 can be connected to the image sensing device 60 via a cable, or the data process device 68 can receive the gray level image and the chromatic image in a wireless transmission way from the image sensing device 60.

Compared to the prior art, the image sensing device and system provided by the invention uses the filter unit array of the new-style color filter and controls the multi-band light and the narrow band light through the filter unit of the color filter to obtain each of band gray level images. Wherein, the gray level image of the red pixel, the green pixel, and the blue pixel can be reconstructed to be a full-band chromatic image, and the narrow band pixel will show the gray level image with high contrast. Therefore, the image sensing device and system of the invention can synchronously or independently show different band images by the outer display device.

Although the present invention has been illustrated and described with reference to the preferred embodiment thereof, it should be understood that it is in no way limited to the details of such embodiment but is capable of numerous modifications within the scope of the appended claims.

The invention provides an image sensing device capturing a gray level image and a chromatic image on a frame via a mixing illumination of a first light and a second light. The image sensing device includes a color filter array module and a controlling module. The color filter array module includes a first filter unit, a second filter unit, a third filter unit, and a fourth filter unit. The first filter unit is used to sense a first pixel data at the frame; the second filter unit is used to sense a second pixel data at the frame; the third filter unit is used to sense a third pixel data at the frame; and the fourth filter unit is used to sense a narrow banding data. The controlling module controls the sensing form of the color filter array module according to the first light or the second light.

## Claims

**1.** An image sensing device, for capturing a gray level image and a chromatic image on a frame via a mixing illumination of a first light and a second light, the image sensing device comprising:

a color filter array module, comprising:

a first filter unit, located in a first region, for sensing a first pixel of the frame;
a second filter unit, located in a second region and near the first region, for sensing a second pixel of the frame;
a third filter unit, located in a third region and near the second region, for sensing a third pixel of the frame; and
a fourth filter unit, located in a fourth region and near the first region and the third region, for sensing a narrow band pixel of the frame; and
a controlling module, coupled to the color filter array module, the first light, and the second light, for controlling a sensing form of the color filter array module according to the first light or the second light.

**2.** The image sensing device of claim 1, wherein the first light is a narrow band light and the second light is a white light.

**3.** The image sensing device of claim 1, wherein when the first light performs exposure, the controlling module starts the fourth filter unit to sense the narrow band pixel and generates the gray level image with high contrast according to the narrow band pixel.

**4.** The image sensing device of claim 3, wherein when the second light performs exposure, the controlling module starts the first filter unit, the second filter unit, and the third filter unit to sense the first pixel, the second pixel, and the third pixel, and generates the chromatic image according to the first pixel, the second

**5.** The image sensing device of claim 1, wherein the first pixel, the second pixel, and the third pixel are correspond to a red pixel, a blue pixel, and a green pixel respectively.

**6.** The image sensing device of claim 1, wherein the fourth filter unit is plated with a specific wavelength film or an electroless plating filter film.

**7.** The image sensing device of claim 1, wherein the image sensing device is a charge coupled device (CCD) or a complementary metal-oxide semiconductor (CMOS).

**8.** An image sensing system, comprising:

an image sensing device, for capturing a gray level image and a chromatic image on a frame, the image sensing device comprising:
a color filter array module, for generating a first pixel, a second pixel, a third pixel, and a fourth pixel;
a luminous module, for generating a multi-band light or a narrow band light; and
a controlling module, coupled to the color filter array module and the luminous module, for controlling a sensing form of the color filter array module according to the multi-band light or the narrow band light by the luminous module; and
a data process device, coupled to the image sensing device, for receiving the first pixel, the second pixel, and the third pixel to reform the chromatic image, and/or receiving the fourth pixel and showing the gray level image.

**9.** The image sensing system of claim 8, wherein the color filter array module

a first filter unit, located in a first region, for sensing a first pixel of the frame;
a second filter unit, located in a second region and near the first region, for sensing a second pixel of the frame;
a third filter unit, located in a third region and near the second region, for sensing a third pixel of the frame; and
a fourth filter unit, located in a fourth region and near the first region and the third region, for sensing a narrow band pixel of the frame.

**10.** The image sensing system of claim 8, wherein when the narrow band light performs exposure, the controlling module starts the fourth filter unit to sense the narrow band pixel and generates the gray level image with high contrast according to the narrow band pixel.

**11.** The image sensing system of claim 8, wherein when the multi-band light performs exposure, the controlling module starts the first filter unit, the second filter unit, and the third filter unit to sense the first pixel, the second pixel, and the third pixel, and generates the chromatic image according to the first pixel, the second pixel, and the third pixel.

**12.** The image sensing system of claim 8, wherein the multi-band light is a white light.

**13.** The image sensing system of claim 8, wherein the first pixel, the second pixel, and the third pixel correspond to a red pixel, a blue pixel, and a green pixel respectively.

**14.** The image sensing system of claim 8, wherein the fourth filter unit is plated with a specific wavelength film or an electroless plating filter film.

**15.** The image sensing system of claim 8, wherein the image sensing device is a charge coupled device (CCD) or a complementary metal-oxide semiconductor (CMOS).

**16.** The image sensing system of claim 8, wherein the data process device is coupled to the image sensing device and used for receiving the gray level image and the chromatic image.

**17.** The image sensing system of claim 8, wherein the data process device receives the gray level image and the chromatic image in a wireless transmission way.

20
R
G
R
G
G
B
G
B
R
G
R
G
G
B
G
B

FIG. 1A

20

Clear Cyan Clear Cyan

Yellow Clear Yellow Clear

Clear Cyan Clear Cyan

Yellow Clear Yellow Clear

FIG. 1B

20

| B | G | R | G |
|---|---|---|---|
| G | IR | G | IR |
| R | G | B | G |
| G | IR | G | IR |

FIG. 1C

20

| R | G | B | C |
|---|---|---|---|
| G | R | C | B |
| B | C | R | G |
| C | B | G | R |

FIG. 1D

4
400
40
402
404
R
B
R
B
406
NB
G
NB
G
R
B
R
B
NB
G
NB
G
46
controlling module
42
44
first light
second light

FIG. 2A

400
40
402
404
G
B
G
B
406
NB
R
NB
R
G
B
G
B
NB
R
NB
R

FIG. 2B

6
60
620
62
622
624
626
R
B
R
B
NB
G
NB
G
R
B
R
B
NB
G
NB
G
66
controlling module
64
luminous module
68

FIG. 3

Europäisches Patentamt
European Patent Office
Office européen des brevets

# EUROPEAN SEARCH REPORT

Application Number
EP 09 17 5523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 1 326 432 A (GIVEN IMAGING LTD [IL]) 9 July 2003 (2003-07-09)<br>col. 2, paragraph 7;<br>col. 4-5, paragraph 26, fig. 1;<br>col. 5, paragraph 26, figs. 1-2;<br>col. 13 , paragraphs 59-60, fig. 6;<br>----- | 1,2,5-9, 12-15,17<br>3,4,10, 11,16 | INV.<br>H04N9/04<br>A61B1/04 |
| X<br>Y | WO 2008/114260 A (GIVEN IMAGING LTD [IL]; PASCAL AMIT [IL]) 25 September 2008 (2008-09-25)<br>page 2, paragraph 4, figs. 1-5C;<br>page 3, pargraph 9;<br>page 4, paragraphs 15-19;<br>page 5, paragraph 21;<br>pages 7-8, paragraphs 26, 28-31;<br>page 9, paragraphs 33-34;<br>----- | 1,2,5-9, 12-15,17<br>3,4,10, 11,16 | |
| Y | EP 2 008 573 A (OLYMPUS MEDICAL SYSTEMS CORP [JP]) 31 December 2008 (2008-12-31)<br>col. 2, paragaphs 8-10;<br>col. 6-7, paragaph 45, fig. 5<br>----- | 3,4,10, 11,16 | TECHNICAL FIELDS SEARCHED (IPC) |
| A | EP 1 715 697 A (GIVEN IMAGING LTD [IL]) 25 October 2006 (2006-10-25)<br>col. 1-2, paragraph 5;<br>col. 3, paragraph 11;<br>col. 5-6, paragraph 17;<br>----- | 1-17 | H04N<br>A61B |

The present search report has been drawn up for all claims

| Place of search | Date of completion of the search | Examiner |
|---|---|---|
| Munich | 19 January 2010 | Dinov, Vassil |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

6

## ANNEX TO THE EUROPEAN SEARCH REPORT ON EUROPEAN PATENT APPLICATION NO. EP 09 17 5523

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2010

| Patent document cited in search report | | Publication date | Patent family member(s) | | Publication date |
|---|---|---|---|---|---|
| EP 1326432 | A | 09-07-2003 | JP | 2003265405 A | 24-09-2003 |
| WO 2008114260 | A | 25-09-2008 | EP | 2124710 A2 | 02-12-2009 |
| | | | US | 2008234548 A1 | 25-09-2008 |
| EP 2008573 | A | 31-12-2008 | CN | 101420901 A | 29-04-2009 |
| | | | WO | 2007123028 A1 | 01-11-2007 |
| | | | KR | 20080102317 A | 24-11-2008 |
| | | | US | 2009041319 A1 | 12-02-2009 |
| EP 1715697 | A | 25-10-2006 | JP | 2006297093 A | 02-11-2006 |
| | | | US | 2006232668 A1 | 19-10-2006 |

EPO FORM P0459

For more details about this annex : see Official Journal of the European Patent Office, No. 12/82